# EUROPEAN PATENT APPLICATION

(11) **EP 3 701 904 A1**
(43) Date of publication of application: **02.09.2020**
(21) Application number: 18830297.0
(22) Date of filing: 26.10.2018
(51) Int. Cl.: A61C 8/00, A61L 27/38

(54) **DENTAL IMPLANT WITH FUNCTIONAL GRADIENT AND ITS PRODUCTION PROCESS**

(30) Priority: 26.10.2017 PT 2017110372
(71) Applicant: Universidade do Minho, 4704-553 Braga (PT)
(72) Inventor: CORREIA PEREIRA SILVA, Filipe Samuel, 4800-058 GUIMARÃES (PT); SOARES MADEIRA, Sara Cristina, 4800-058 GUIMARÃES (PT); SALGADO PINTO, Paulo Filipe, 4800-058 GUIMARÃES (PT); NOVAIS CARVALHO, Óscar Samuel, 4800-058 GUIMARÃES (PT)
(74) Representative: Patentree
(86) International application number: PCT/IB2018/058404
(87) International publication number: WO 2019/082160

(57) **Abstract**

The present disclosure describes a dental implant with functional gradient and respective obtention method, wherein the implant comprises an inner part of metal or metal alloy or titanium-based metal matrix composite which comprises on its surface a transition zone of rugose texture or surface pattern; an outer part composed of a ceramic or zirconia-based ceramic composite, and a diffusion protective film between the transition zone and the outer part described as an oxide and/or nitride protective film.

## Description

### Technical domain

The present disclosure is in the dental area, more specifically in the area of dental implants and refers to an implant whose inner part is metallic and the outer part is ceramic.

### Background of the invention

The components which are composed of an inner part of a metallic material and an outer part of a ceramic material, are usually obtained using coating technologies of the inner part. Coatings are common, obtained by plasma spray [US 20060052880 A1], flame spray [US 4645716 A], ion bombardment [US 20060233944 A1], among other coating methods.

Another way of obtaining the union of two zones, internal and external, with different materials, involves sintering of powders, from a metallic interior and a ceramic exterior, with a gradient between the zones that will constitute an internal zone based on titanium or its alloys, a transition zone consisting of a titanium composite or its alloys and zirconia or mixture of zirconia and alumina, and the outer zone consisting of zirconia powders or a mixture of zirconia and alumina, other ceramics such as hydroxyapatite, among others [WO2013 / 043039] may further be included. This distribution of powders will be sintered by HP-Hot Pressing, HIP-Hot Isostatic Pressing, SPS-Spark Plasma Sintering, or other method where temperature and pressure are applied, until consolidation of the powders and obtention of the final part. In the case of the former, obtained with various coatings, adhesion between the inner and outer parts is normally low and phenomena such as delamination or separation between the parts are often reported, when the components are in service [Sun L, Bernd CC, Gross KA, Kucuk A. Material fundamentals and clinical performance of plasma sprayed hydroxyapatite coatings: a review. Journal of biomedical materials research 2001; 58: 570-92].

In the case of powder sintering, where the different materials are sintered simultaneously, there are problems associated with the diffusion between the different materials, which lead to the creation of fragile zones as well as strong aesthetic alterations, with color change of zirconia, [Hideaki Tsukamoto, Mechanical properties of zirconia-titanium composites, International Journal of Materials Science and Applications 2014; 3 (5): 260-267 and Kun-Lin Lin and Chien-Cheng Lin, Reaction Between Titanium and Zirconia Powders During Sintering at 1500 ° C, J. Am. Ceram. Soc., 90 (7) 2220-2225 (2007)].

These facts are described in order to illustrate the technical problem solved by the embodiments of the present document.

### General description

The way of obtaining a component with the two parts, an inner metallic and an outer ceramic part, with a transition zone that allows high interface and overall properties and maintaining the aesthetic properties of zirconia, is proposed in this document.

The present disclosure relates to a dental implant, metal-ceramic, with an inner part or core, formed by a metal or metal alloy or titanium-based metal matrix composite, which is tough and resistant to fracture; and an outer part composed of a ceramic or ceramic composite, biocompatible, and with an aesthetic function, possibly bioactive, based on zirconia containing yttria or ceria or magnesia, among other materials that stabilize zirconia, and optionally alumina or bioactive and/or antibacterial materials such as hydroxyapatite, βTCP, or bioglass. A physical transition zone is created between the two parts, with rugose texture or surface pattern that allows adequate adhesion and stress distribution, aiming to increase the mechanical and fracture properties of the interface between the core and the outer part; and a barrier to the diffusion between the materials of the inner and outer parts.

In one embodiment, the process for obtaining the component is based on the following approach: preparation of a solid metal rod in Titanium (Ti) or its alloys, with a texturizing surface treatment, by mechanical, physical and/or chemical route, a treatment that gives rise to the transition zone with the outer ceramic part; followed by a treatment of creating a surface film, by oxidation, nitriding or oxy-nitriding on the same metal rod, for the creation of a diffusion barrier between the materials of the titanium rod and the exterior based on zirconia; followed by the placement of the solid metal rod, which will constitute the inner part of the implant, in a mould made of graphite or a refractory material, leaving a free zone between the rod and the mould; followed by placement of ceramic powders in this zone between the rod and the mould, which will give rise to the external layer of the implant. After placing the materials that will constitute the implant, namely the solid metal rod, already with a surface film, and the ceramic powders, these will be pressed and sintered under pressure and temperature, to consolidation of the component and to obtain the final or near final geometry, of the implant.

In one embodiment, one advantage of the implant of the present disclosure is that the implant simultaneously has a high fracture toughness, essentially imparted by the metallic interior, and also has an outer ceramic part. A further advantage of this component is that it has a good adhesion between the inner metallic part and the outer ceramic part, due to the surface texture or treatment that will allow a mechanical entrapment between the two materials, of the interior, metallic, and of the outer layer, ceramic. Yet another advantage is that the outer part, based on zirconia, maintains the aesthetic characteristics in terms of color, namely being approximately white (zirconia color) due to the barrier to diffusion, created on the inner metallic part. Yet another advantage is that the outer part, based on zirconia, is biocompatible (because zirconia is biocompatible), and possibly bioactive and antibacterial because it is possible to add to zirconia bioactive and antibacterial materials such as hydroxyapatite, βTCP, bioglass, among other bioactive and/or antibacterial materials.

A further aspect of the present disclosure is still a method for the production of the implant described in the present disclosure. In one embodiment, the inner metallic part, and the outer ceramic part, are consolidated using hot-pressing technology or similar (spark-plasma-sintering) in which one of the parts, the metallic part, is initially already in the solid state and the other part is initially powdered. The powder is consolidated onto the surface of the inner solid part using pressure and temperature, after preparation of the solid interior surface using a mechanical, physical and/or chemical treatment. Thus, another advantage of the present disclosure is that the final implant is obtained by hot pressing the powdered part on the inner solid part.

The functional gradient is a gradation of different materials.

The transition zone is a zone intended to increase the mechanical and fracture properties of the interface between the core and the outer part.

The chemical transition barrier (or diffusion barrier) is a protective film which is intended to create a barrier to the diffusion between the materials of the outer ceramic part and the inner metallic part during processing.

A further aspect of the present disclosure describes a dental implant which comprises:
metallic interior of the implant with an external surface wherein the external surface is rugose or has recesses,
protective film on said external surface wherein the protective film comprises titanium oxide, nitride, and/or oxy-nitride, or combinations thereof, as a barrier to diffusion between the interior and exterior of the implant;
exterior of the implant over said protective film. Implant according to previous claim wherein the rugosity or recesses of the external surface of the metallic interior of the implant cause corresponding rugosity or recesses in said protective film.

In one embodiment, the external rugose surface or the recesses of the metallic interior have a depth of 1 micrometer to 1, 5 millimeters.

In one embodiment, the rugosity or recesses of the external surface of the metallic interior of the implant have a pattern of grooves.

In one embodiment, the grooves intersect at an angle of 90°.

In one embodiment, the metallic interior of the implant is a metal, a metal alloy, a metal matrix composite.

In one embodiment, the metallic interior of the implant comprises titanium and/or the exterior of the implant comprises zirconia.

In one embodiment, the grooves have a depth between 0.1 and 1.5 millimeters, preferably 0.5 millimeters.

In one embodiment, the rugosity or recesses of the external surface of the metallic interior of the implant has a thickness between 1 micrometer and 1.5 millimeters (in that the rugosity can be measured using equipment for surface geometric measurements).

In one embodiment, the protective film is of titanium oxide and/or nitride or titanium oxi-nitride.

In one embodiment, the protective film has a thickness between 2 nanometers and 20 micrometers.

In one embodiment, the metallic interior of the implant has a diameter between 1.5 and 8 millimeters.

In one embodiment, the metallic interior has a fracture toughness ranging from 80 to 120 MPam^{(1/2)}.

In one embodiment, the outer part is comprised of zirconia containing at least one of the following list elements: yttria, ceria, CaO, MgO alumina, or their mixtures.

In one embodiment, the amount of yttria on the exterior of the implant ranges from 2 to 10% by weight.

In one embodiment, the amount of ceria on the exterior of the implant ranges from 1 to 20% by weight.

In one embodiment, wherein the amount of alumina on the exterior of the implant is up to 20% by weight.

In one embodiment, the outer part of the implant comprises: hydroxyapatite, βTCP, bioglass, or combinations thereof. In particular, the amount of hydroxyapatite, βTCP, bioglass, or combinations thereof is at most 50% by volume.

In one embodiment, the bioglass comprises at least one of the following compounds: compounds comprising silica, compounds comprising SiO2, compounds comprising calcium oxide, compounds comprising CaO, compounds comprising sodium oxide, compounds comprising Na2O, or mixtures thereof.

In one embodiment, the outer part has a thickness ranging from 0.1 to 1.5 millimeters.

A further aspect of the present invention describes a process of obtaining the implant described in any one of the preceding claims comprising the following steps:
applying a mechanical, physical and/or chemical treatment to a titanium rod so as to give rise to the one external surface wherein the external surface is rugose or has recesses;
obtaining a film, over the rugose external surface or recesses, preferably by chemical, electrochemical, physical route using plasma depositions, temperature route, or combinations thereof;
placing the metal rod with external surface wherein the external surface is rugose or has recesses and protective film inside the body of the mould;
adding ceramic powders in the space between the rod and the interior of the mould in which the powders preferably comprise zirconia;
adding the upper part of the mould (2);
heating the assembly to a temperature between 900 ° C and 1600 ° C, preferably at 1180 ° C, in an ambient under vacuum and/or controlled atmosphere;
during heating applying a pressure on the powders between 5MPa and 200MPa, preferably 60MPa;
after 5 to 60 minutes, preferably 15 minutes, withdrawing the pressure and allow to cool down to room temperature.

In one embodiment, the dental implant comprises: an inner part of metal or metal alloy or titanium-based metal matrix composite, comprising on its surface a physical transition zone, with rugose texture or surface pattern; an outer part composed of a ceramic or ceramic composite, based on zirconia; a barrier between the transition zone of the inner metallic part and the outer ceramic part, which consists in a protective film of oxide or nitride or both or oxy-nitride.

In one embodiment, the implant has a transition zone having rugosity, preferably having a pattern of grooves, where the grooves preferably intersect at 90º and have a preferred depth of about 0.5 mm.

In one embodiment, the transition zone has a thickness between 1 micrometer and 1,5 millimeters.

In one embodiment, the diffusion barrier is preferably of titanium oxide and/or nitride or titanium oxy-nitride.

In one embodiment, the diffusion barrier has a thickness between 2 nanometers and 20 microns.

In one embodiment, the inner part is of titanium or titanium alloy, pure or of any grade, or titanium-based metal matrix composite, has a diameter between 1.5 and 8 mm and has a fracture toughness between 80 and 120 MPam^(1/2).

In one embodiment, the outer part comprises zirconia with yttria or ceria or CaO or MgO or alumina, wherein the amount of yttria ranges from 2 to 10% by weight; the amount of ceria ranges from 1 to 20% by weight and the amount of alumina is up to 20% by weight.

In one embodiment, the outer part optionally comprises hydroxyapatite or βTCP or bioglass (compounds based on silica, SiO2, Calcium oxide, CaO, Sodium oxide, Na2O, among others), in percentages that may reach up to 50% by volume and by having a thickness between 0.1 and 1.5 millimeters.

In one embodiment, the method of obtaining the implant comprises the following steps:
in a titanium rod performing a mechanical, physical and/or chemical treatment, so as to give rise to the surface texture;
producing the diffusion protective film, over the texture, by chemical or electrochemical or physical route using plasma depositions, or even by temperature route;
placing the metal rod inside the mould body (1), resting on the base of the mould (3), which in the meantime is fixed to the main body of the mould (1), thus leaving a space between the rod (4) and the interior of the mould;
placing ceramic powders in the space between the rod (4), and the interior of the mould, the powders preferably comprising zirconia;
after placing the powders, the upper part of the mould (2) is placed;
the whole assembly is heated to a temperature between 900 ° C and 1600 ° C, preferably at 1180 ° C, in an ambient under vacuum and/or controlled atmosphere;
during the heating process pressure (6) is applied to the powders, from the lower (3) and upper (2) parts of the mould, between 5MPa and 200MPa, preferably 60MPa;
after 5 to 60 minutes, preferably 15 minutes, withdrawing the pressure and leaving to cool down to room temperature.

In one embodiment, the described process is characterized in that the mechanical, physical or chemical, treatment for the creation of the surface texture, consists of the projection of ceramic particles, by an acid treatment, by a laser ablation treatment, or by mechanical machining.

In one embodiment, the described process is characterized in that the production of the diffusion protective film by electrochemical oxidation have electrical potentials preferably between 80 and 120V using as electrolyte preferably phosphoric acid (H3PO4) and/or sulfuric acid (H2SO4).

In one embodiment, the described process is characterized in that the production of the diffusion protective film by oxidation with temperature uses temperatures between 200 ° C and 1200 ° C, in air or in an oxygen enriched environment, and with exposures from a few minutes to several days.

### Brief description of the drawings

For an easier understanding, the following drawings are attached, which represent preferred embodiments which are not intended to limit the object of the present description.

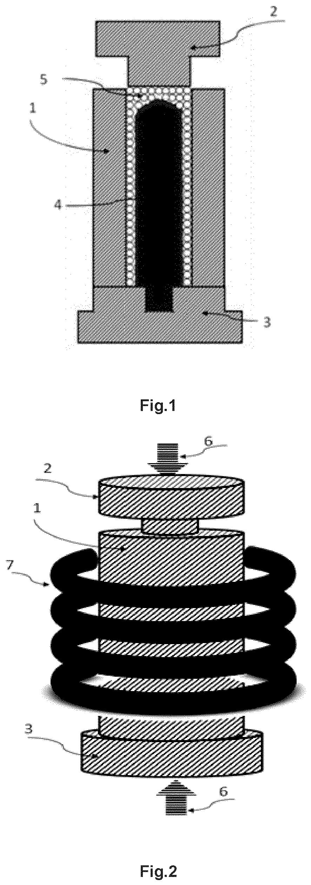

**Figure 1** **-** Embodiment of a mould with the main body (1), the upper part (2) and the lower part (3) into which the titanium rod (4) and the zirconia-based powders (5) are placed.
**Figure 2** - Embodiment of application of pressure (6) and temperature (7), for implant consolidation, in which the pressure is applied on the upper part (2) and on the lower part (3) of the mould. Inside the mould there is the metal rod (4) already with treated surface (10) and the ceramic powders (5).

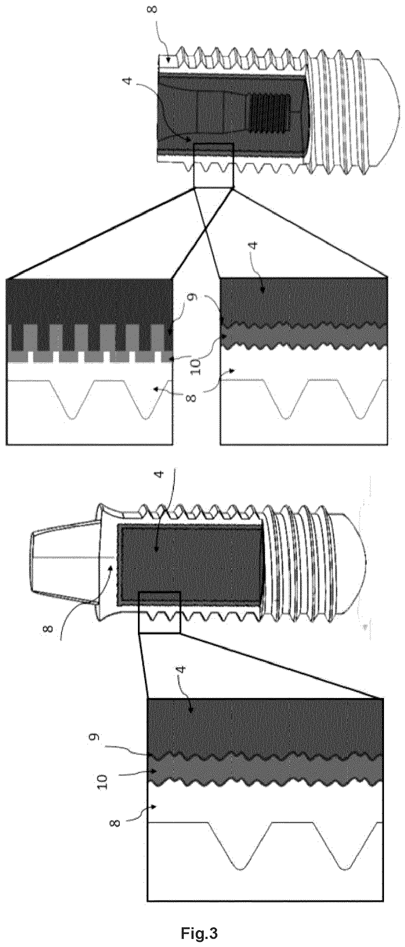

**Figure 3** - Embodiment of dental implants where it can be seen the titanium-based inner metallic part (4), the zirconia-based outer ceramic part (8), the physical transition zone (9) between the inner metallic part and the outer ceramic part, and the diffusion protective film of oxide, nitride or oxy-nitride (10).

### Detailed Description

The present disclosure describes a dental implant, which is composed of an inner part or core, formed by a metal or metal alloy or titanium-based metal matrix composite, tough and resistant to fracture; by a transition zone which will provide a high mechanical connection between the inner metallic part and the outer ceramic part; and by a film that will prevent the diffusion of materials between them during processing; and by an outer part composed of a ceramic or ceramic composite, biocompatible, with aesthetic function and possibly bioactive, based on zirconia, optionally containing alumina, hydroxyapatite, βTCP, bioglass, among other zirconia stabilizing materials and/or bioactive and/or antibacterial.

This disclosure further relates to a method of producing the dental implant in which the inner, metallic component, and the outer, ceramic component, are consolidated using hot-pressing technology, or spark-plasma-sintering, or equivalent, and wherein the metallic part is already in the solid rod state, and the ceramic part is in powder. The powder is consolidated on the surface of the solid part using pressure and temperature, after preparation of the solid surface, using a mechanical, physical, thermal and/or chemical treatment.

In one embodiment, according to Figure 3 the dental implant comprises an inner metallic part (4). This part comprises pure titanium or any other grade, or by a titanium alloy, for example Ti6AI4V, or by a titanium-based metal matrix composite, for example Ti6AI4V containing 0.1% to 5% by volume of AI203, or other reinforcement. This metallic interior should have the fracture toughness characteristics of titanium or its alloys, for example between 80 and 120 MPam^{(1/2)}, and has a diameter ranging from 1.5 to 8 millimeters.

In one embodiment, the physical transition zone (9), created on the inner metallic part is a surface texture or pattern, which will create a strong mechanical connection between the metallic interior and the ceramic exterior, and which has a thickness which can range from 1 micrometer to 1.5 millimeters. This zone, which may consist of some rugosity but will preferably be by a pattern of grooves made by laser ablation, or CNC machining, on the surface of the inner metal rod, with a depth that can reach up to 1,5 millimeters, is intended to create a mechanical interlocking between the material of the inner part and the material of the outer part, which will penetrate the grooves (or texture) when pressure and temperature are applied.

In one embodiment, the chemical transition barrier (10) consists of a diffusion protective film formed by a titanium oxide and/or nitride or titanium oxi-nitride and which is intended to create a barrier to the diffusion between the materials of the outer ceramic part and of the inner metallic part during processing, and may have a thickness ranging from 2 nanometers to 20 micrometers. This layer is applied to the metallic part after the physical transition zone has been created.

In one embodiment, the outer part (8) comprises zirconia, which is stabilized with yttria (ranging from 2 to 10% by weight), ceria (from 1 to 20% by weight), or other zirconia stabilizer such as CaO, MgO, among others, and may still contain alumina in percentages up to about 20% by weight.

In one embodiment, the implant further comprises an outer part, zirconia based, which may contain bioactive or antibacterial materials such as hydroxyapatite, beta tricalcium phosphate (βTCP), or Bioglass (compounds based on silica, SiO2, Calcium oxide, CaO, Sodium oxide, Na2O, among others), in percentages that may reach up to 50% by volume. The outer part must have a thickness that can range from about 0.1 mm to about 1.5 mm.

In one embodiment, the implant of the present disclosure has fracture toughness which comes essentially from the metallic interior, and the biocompatibility; and aesthetics that come from the zirconia exterior, and may be as well bioactive and/or antibacterial through the incorporation of bioactive and/or antibacterial materials on the zirconia-based outer part.

In one embodiment, according to Figure 1, the implant is processed within a mould that may comprise the central body (1), the upper part (2), and lower part (3). This mould may be in graphite or in a refractory material such as Tungsten, Niobium, Molybdenum, among others.

In one embodiment, the implant of the present disclosure may be obtained from a titanium-based metal rod (4) and may be of pure titanium of any grade, or an alloy of titanium, or of a titanium matrix composite, preferably a Ti6AI4V alloy, and surface treated with a mechanical, physical and/or chemical treatment, in order to give rise to a surface texture on the titanium rod (9), which will serve to promote a strong mechanical connection between the parts and create a transition zone between the metal and the outer ceramic.

In a transition zone, i.e. the external surface wherein the external surface is rugose or has recesses, consisting of a surface texture, can be obtained by projection of ceramic particles, by an acid treatment, using for example sulfuric acid, by a laser ablation treatment, or by mechanical machining, where textures as holes or grooves are created, with certain depth and pattern, and which are intended to create a good mechanical connection between the inner metallic layer and the outer ceramic layer. It should preferably be created a pattern of grooves, with grooves that intersect at 90 °, by laser ablation or by CNC machining, with a depth of about 0.5 mm. To the transition zone there may further be added a diffusion protective film (10) of oxide, nitride, or oxy-nitride, obtained by conventional chemical or electrochemical route, or physical, also conventional, by plasma depositions, or even by temperature route, also conventional, which is intended to avoid diffusion between the elements of the inner metallic part and of the outer ceramic part during processing.

Preferably an electrochemical oxidation should be carried out. As an example of electrochemical oxidation there may be mentioned electric potential parameters between 80 and 120V using as electrolyte phosphoric acid (H3PO4) and/or sulfuric acid (H2SO4), giving rise to dense thin films. As an example of oxidation with temperature there may be mentioned temperatures between 200 ° C and 1200 ° C, in the air or in an oxygen enriched environment, and with exposures of some minutes to several days. This diffusion protective film is made over the texture previously performed.

In one embodiment, in the implant of the present disclosure, the metal rod (4), after being treated superficially, with texture and with protective film, is placed inside the mould body (1), resting on the base of the mould (3) which, however, is secured to the main body of the mould (1), with an existing space between the rod (4) and the interior of the mould. In this space the ceramic powders (5) will be placed. Ceramic powders will be essentially based on zirconia and may contain other elements as described above. Preferably it may be zirconia stabilized with 3% (volume) of yttria.

After the powders are placed, the upper part of the mould (2) is placed, and the assembly is heated (7) at a temperature between 900 ° C and 1600 ° C, preferably at 1180 ° C. The heating should preferably take place in an ambient under vacuum and/or controlled atmosphere, for example with argon, to prevent oxidation of the titanium. During the heating process pressure (6) is applied to the powders from the lower (3) and upper (2) parts of the mould, up to values which may oscillate between 5MPa and 200MPa, preferably 60 MPa.

After a period of time, which may range from 5 minutes to 60 minutes, preferably 15 minutes, wherein the powders are subjected to the indicated pressure and temperature, pressure is withdrawn and the temperature will naturally decrease to room temperature.

In one embodiment, the implant of the present disclosure shown in Figure 3 illustrates the implant in near-final shape, in which there is the inner part (4), metallic, and the outer part (8), ceramic, and a zone of the physical transition (9) between the two parts and a diffusion protective film (10). The outer geometry of the zirconia part may already contain the final geometry of the implant, including thread or other texture.

In one embodiment, the implant of the present disclosure has a fracture toughness that comes essentially from the metallic interior and the biocompatibility and aesthetics that comes from the exterior in zirconia, and can also be bioactive and/or antimicrobial by incorporating bioactive and/or antibacterial materials into the zirconia. It also has a high mechanical connection between the inner metallic part and the outer ceramic part and will maintain the aesthetic part of the zirconia.

The term "comprises" or "comprising" when used in this document is intended to indicate the presence of the characteristics, elements, integers, steps and components mentioned, but does not prevent the presence or addition of one or further features, elements, integers, steps and components, or groups of the same.

The present disclosure is not, of course, in any way restricted to the described embodiments in this document and a person of ordinary skill in the art may foresee many possibilities of modifying it and replacing technical characteristics by equivalent ones, depending on the requirements of each situation, as defined in the appended claims.

The following claims further define preferred embodiments.

## Claims

1. Dental implant comprising:
metallic interior of the implant with an external surface wherein the external surface is rugose or has recesses;
protective film on said external surface wherein the protective film comprises titanium oxide, nitride, and/or oxy-nitride, or combinations thereof as a barrier to the diffusion between the interior and exterior of the implant; exterior of the implant over said protective film.

2. Implant according to the preceding claim, wherein the rugosity or recesses of the external surface of the metallic interior of the implant have corresponding rugosity or recesses in said protective film.

3. Implant according to any one of the preceding claims, wherein the rugose external surface or the recesses of the metallic interior have a depth of 1 micrometer to 1, 5 millimeters.

4. Implant according to any one of the preceding claims, wherein the rugosity or the recesses of the external surface of the metallic interior of the implant has a pattern of grooves.

5. Implant according to any one of the preceding claims, wherein the grooves intersect with a 90 ° angle.

6. Implant according to any one of the preceding claims, wherein the metallic interior of the implant is a metal, a metal alloy, a metal matrix composite.

7. Implant according to any one of the preceding claims, wherein the metallic interior of the implant comprises titanium.

8. Implant according to any one of the preceding claims, wherein the exterior of the implant comprises zirconia.

9. Implant according to any one of the preceding claims, wherein the grooves have a depth of between 0.1 and 1.5 millimeters, preferably of 0.5 millimeters.

10. Implant according to any one of the preceding claims, wherein the rugosity or the recesses of the external surface of the metallic interior of the implant has a thickness between 1 micrometer and 1.5 millimeters.

11. Implant according to any one of the preceding claims, wherein the protective film is of titanium oxide and/or nitride or titanium oxy-nitride.

12. Implant according to the preceding claim, wherein the protective film has a thickness between 2 nanometers and 20 micrometers.

13. Implant according to any one of the preceding claims, wherein the metallic interior of the implant has a diameter between 1.5 and 8 millimeters.

14. Implant according to any one of the preceding claims, wherein the metallic interior has a fracture toughness ranging from 80 to 120 MPam^{(1/2)}.

15. Implant according to any one of the preceding claims, wherein the outer part is comprised of zirconia containing at least one of the elements of the following list: yttria, ceria, CaO, MgO alumina, or mixtures thereof.

16. Implant according to the preceding claim, wherein the amount of yttria on the exterior of the implant ranges from 2 to 10% by weight.

17. Implant according to claim 15, wherein the amount of ceria on the exterior of the implant ranges from 1 to 20% by weight.

18. Implant according to claim 15, wherein the amount of alumina on the exterior of the implant is up to 20% by weight.

19. Implant according to any one of the preceding claims, wherein the outer part of the implant comprises: hydroxyapatite, βTCP, bioglass, or combinations thereof.

20. Implant according to the preceding claim, wherein the amount of hydroxyapatite, βTCP, bioglass, or combinations thereof is at most 50% by volume.

21. Implant according to the preceding claim, wherein the bioglass comprises at least one of the following compounds: compounds comprising silica, compounds comprising SiO2, compounds comprising calcium oxide, compounds comprising CaO, compounds comprising sodium oxide, compounds comprising Na2O, or their mixtures.

22. Implant according to any one of the preceding claims, wherein the outer part has a thickness ranging from 0.1 to 1.5 millimeters.

23. Process of obtaining the implant described in any one of preceding claims which comprises the following steps:
applying a mechanical, physical and/or chemical treatment to a titanium rod so as to originate the one external surface wherein the external surface is rugose or has recesses;
obtaining a film on the rugose external surface or recesses, preferably by chemical, electrochemical, physical route using plasma depositions, temperature route, or combinations thereof;
placing the metal rod with external surface wherein the external surface is rugose or has recesses and protective film inside the body of the mould;
adding ceramic powders in the space between the rod and the interior of the mould, the powders preferably comprising zirconia;
adding the upper part of the mould (2);
heating the assembly to a temperature between 900 ° C and 1600 ° C, preferably at 1180 ° C, in an ambient under vacuum and/or controlled atmosphere;
during heating applying a pressure on the powders between 5MPa and 200MPa, preferably 60MPa;
after 5 to 60 minutes, preferably 15 minutes, withdrawing the pressure and allow to cool down to room temperature.

24. Process according to the preceding claim, wherein the external surface wherein the external surface is rugose or has recesses comprises the projection of ceramic particles, by an acid treatment, by a laser ablation treatment, by mechanical machining, or combinations thereof.

25. Process according to any one of claims 23-24, wherein the protective film is obtained by electrochemical oxidation with electrical potentials; preferably between 80 - 120V using as electrolyte preferably phosphoric acid (H3 PO4) and/or sulfuric acid (H2 SO4).

26. Process according to any one of claims 23-25, wherein the protective film is obtained diffusion by oxidation with temperatures between 200 ° C and 1200 ° C, in the air or in an oxygen enriched environment, and with exposures from a few minutes to several days.
